(19) Europäisches Patentamt
European Patent Office
Office européen des brevets

(11) Veröffentlichungsnummer: **0 043 490**
**B1**

(12) **EUROPÄISCHE PATENTSCHRIFT**

(45) Veröffentlichungstag der Patentschrift: **28.09.83**

(51) Int. Cl.³: **C 07 C 120/00** // C07D253/06

(21) Anmeldenummer: **81104822.2**

(22) Anmeldetag: **23.06.81**

(54) **Verfahren zur Herstellung von Acylcyaniden.**

(30) Priorität: **04.07.80 DE 3025304**

(43) Veröffentlichungstag der Anmeldung: **13.01.82 Patentblatt 82/2**

(45) Bekanntmachung des Hinweises auf die Patenterteilung: **28.09.83 Patentblatt 83/39**

(84) Benannte Vertragsstaaten: **BE CH DE FR GB IT LI NL**

(56) Entgegenhaltungen:
**EP-A-0 005 484**
**DE-A-2 614 240**
**DE-A-2 614 241**
**GB-A-583 646**
**BULLETIN OF THE CHEMICAL SOCIETY JAPAN, Band 52, Nr. 10, Oktober 1979, Seiten 2966—2969, Tokyo, JP. AKIRA OKU et al.: »Acyl cyanide. V. The synthesis of 1-cyano-1-alkenyl esters by the reaction of acyl cyanides with acid anhydrides and isocyanates«**

(73) Patentinhaber: **BAYER AG, Zentralbereich Patente, Marken und Lizenzen, D-5090 Leverkusen 1, Bayerwerk (DE)**

(72) Erfinder: **Findeisen, Kurt, Dr., In der Follmuehle 10, D-5068 Odenthal 2 (DE)**
Erfinder: **Krätzer, Hans, Dr., Am Acker 17, D-5600 Wuppertal 1 (DE)**
Erfinder: **Lenthe, Manfred, Dr., Michaelshoehe 40, D-5068 Odenthal 2 (DE)**

**EP 0 043 490 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Acylcyaniden

Die vorliegende Erfindung betrifft ein Verfahren zur Herstellung von Acylcyaniden durch Umsetzung von Carbonsäureanhydriden mit $\alpha$-Hydroxynitrilen. Die Acylcyanide sind weitgehend bekannt und können als Ausgangsstoffe zur Synthese von Herbiziden verwendet werden.

Es ist seit langem bekannt, Carbonsäureanhydride mit Blausäure bei hohen Temperaturen in der Gasphase in Gegenwart bestimmter Katalysatoren zu Acylcyaniden umzusetzen (vgl. GB-PS 583 646). Der Hauptnachteil dieses Verfahrens liegt darin, daß es zu geringe Ausbeuten liefert; es ist daher als technisches Verfahren nicht von Interesse.

Weiterhin ist bekannt, daß man Acylcyanide dadurch erhalten kann, daß man Carbonsäureanhydride mit Alkalicyaniden oder flüssiger wasserfreier Blausäure, gegebenenfalls in Gegenwart eines Verdünnungsmittels, bei Temperaturen zwischen 50 und 250°C umsetzt und die entstandenen Acylcyanide unmittelbar nach ihrer Bildung durch Destillation aus dem Reaktionsmedium entfernt (vgl. DE-OS 2 614 241) oder dadurch, daß man Carbonsäureanhydride in Gegenwart des entsprechenden Carbonsäurechlorids mit Alkalicyanid oder flüssiger wasserfreier Blausäure, gegebenenfalls in Gegenwart eines Verdünnungsmittels, bei Temperaturen zwischen 50 und 300°C umsetzt (vgl. DE-OS 2 614 240). Bei beiden vorgenannten Verfahren ist ein Nachteil darin zu sehen, daß nur mäßige Ausbeuten an aliphatischen Acylnitrilen erhalten werden; so läßt sich beispielsweise Pivaloylcyanid in Ausbeuten von 71 bzw. 67% der Tehorie herstellen.

Es wurde nun überraschend gefunden, daß man die teilweise bekannten Acylcyanide der allgemeinen Formel

$$R^1-CO-CN \qquad (I)$$

in welcher

$R^1$ für geradkettiges oder verzweigtes Alkyl mit 1–4 C-Atomen, welches durch Alkoxy mit 1–4 C-Atomen, Carbalkoxy mit 1–4 C-Atomen in der Alkoxygruppe, Nitro, Cyano und/oder Halogen (wie Fluor, Chlor, Brom oder Jod) substituiert sein kann; für Cycloalkyl mit 5–6 Ring-C-Atomen, welches durch Alkyl, Alkoxy oder Carbalkoxy mit jeweils bis zu 4 C-Atomen, Nitro, Cyano und/oder Halogen (wie Fluor, Chlor oder Brom) substituiert sein kann; für Aryl, insbesondere Phenyl oder Naphthyl, welches durch Alkyl, Alkoxy oder Carbalkoxy mit jeweils bis zu 4 C-Atomen, Nitro und/oder Halogen (wie Fluor, Chlor oder Brom) substituiert sein kann, oder für 5- oder 6gliedrige heterocyclische Reste steht, die 1 bis 3 Heteroatome wie Sauerstoff, Schwefel und/oder Stickstoff im Ring enthalten können und außerdem mit einem Benzolring anelliert sein können,

in hohen Ausbeuten und in hoher Reinheit erhält, wenn man Carbonsäureanhydride der allgemeinen Formel

$$R^1-CO-O-CO-R^1 \qquad (II)$$

in welcher

$R^1$ die oben angegebene Bedeutung hat,

mit $\alpha$-Hydroxynitrilen der allgemeinen Formel

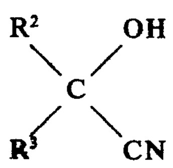

(III)

in welcher

$R^2$ und $R^3$ gleich oder verschieden sein können und für geradkettiges oder verzweigtes Alkyl mit 1–8 C-Atomen, für Cycloalkyl mit 5 oder 6 Ring-C-Atomen, welches durch Alkyl oder Carbalkoxy mit jeweils 1–5 C-Atomen substituiert sein kann, oder für Phenyl oder Naphthyl stehen, welches jeweils durch Alkyl, Halogenalkyl, Alkoxy oder Carbalkoxy mit jeweils bis zu 4 C-Atomen, Nitro und/oder Halogen (wie Fluor, Chlor oder Brom) substituiert sein kann,

gegebenenfalls in Gegenwart eines Verdünnungsmittels bei Temperaturen zwischen 250° und 600°C umsetzt.

Es ist als ausgesprochen überraschend zu bezeichnen, daß Acylcyanide der Formel (I) nach dem erfindungsgemäßen Verfahren in hoher Ausbeute und ausgezeichneter Reinheit zugänglich sind, denn im Hinblick auf den bekannten Stand der Technik war zu erwarten, daß als Hauptreaktion — im Sinne folgender Reaktionsgleichungen — entweder nach (a) acylierte Cyanhydrine oder nach (b) durch deren pyrolytischen Abbau ungesättigte Nitrile (vgl. zum Beispiel V. Migrdichian, »The Chemistry of Organic Cyanogen Compounds«, Reinhold Publishing Corp., New York, 1947, S. 190) bzw. deren Folgeprodukte, z. B. Polymere, gebildet werden:

(a)

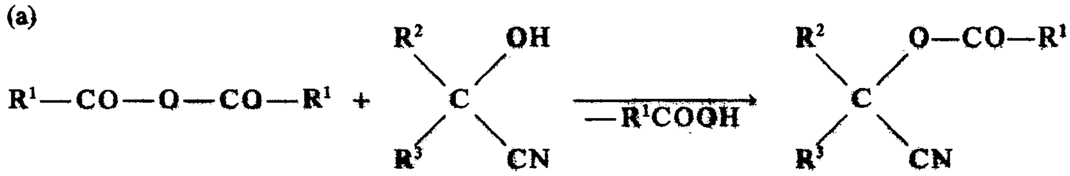


(b) zum Beispiel:

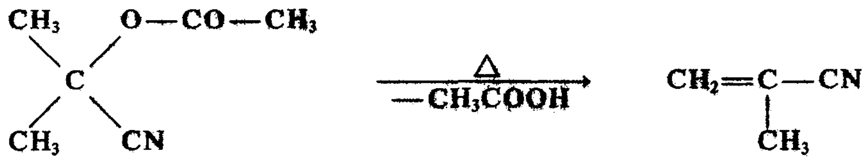


Ein besonderer Vorteil gegenüber den vorbekannten Verfahren besteht darin, daß das erfindungsgemäße Verfahren hohe Ausbeuten auch an aliphatischen Acylcyaniden liefert. Ein zusätzlicher Vorteil gegenüber dem aus GB-PS 583 646 bekannten katalytischen Verfahren ist darin zu sehen, daß das erfindungsgemäße Verfahren keinen Katalysator benötigt. Außerdem ist hervorzuheben, daß bei dem erfindungsgemäßen Verfahren die Aufarbeitung keine Probleme bietet: die Reaktionsprodukte können im allgemeinen leicht durch Destillation oder Filtration getrennt werden; die Nebenprodukte der Umsetzung, Carbonsäuren und Ketone bzw. Aldehyde, können nach bekannten Verfahren wieder in die Ausgangsprodukte, Carbonsäureanhydride bzw. Cyanhydrine, rückverwandelt werden.

Verwendet man Pivalinsäureanhydrid und Acetoncyanhydrin als Ausgangsstoffe, so kann der Reaktionsverlauf durch das folgende Formelschema wiedergegeben werden:

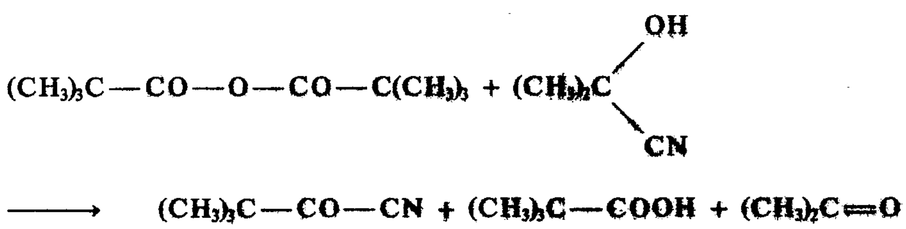

Die als Ausgangsstoffe verwendeten Carbonsäureanhydride der Formel (II) sind bekannt oder können nach an sich bekannten Verfahren hergestellt werden (vgl. zum Beispiel Houben – Weyl, Methoden der Organischen Chemie, 4. Auflage, Band VIII, Seiten 476 – 480 [1952]).

Als Beispiele für insbesondere für $R^1$ in Frage kommende heterocyclische Reste seien genannt: Morpholinyl, Imidazolyl, Pyrazolyl, Pyrrolyl, Isoxazolyl, Piperidinyl, Oxazolyl, 1,2,4-Triazol-1-yl, 1,2,4-Triazol-4-yl, 1,2,3-Triazolyl, 1,2,4-Thiadiazol-2-yl, Benzimidazolyl und Furanyl.

Als bevorzugte Beispiele für Carbonsäureanhydride der Formel (II) seien im einzelnen genannt: Essigsäureanhydrid, Propionsäureanhydrid, Pivalinsäureanhydrid, Cyclohexancarbonsäureanhydrid, Benzoesäureanhydrid, m-Chlorbenzoesäureanhydrid, 3,5-Dichlorbenzoesäureanhydrid, Naphthalin-1-carbonsäureanhydrid, 1-Phenyl-5-pyrazolon-3-carbonsäureanhydrid. Als besonders bevorzugte Anhydride seien Pivalinsäureanhydrid sowie die aromatischen Carbonsäureanhydride, aus dieser Gruppe insbesondere das Benzoesäureanhydrid, genannt.

Die weiterhin als Ausgangsstoffe verwendeten α-Hydroxynitrile (Cyanhydrine) der Formel (III) sind bekannt oder können nach an sich bekannten Verfahren hergestellt werden (vgl. zum Beispiel Houben – Weyl, Methoden der Organischen Chemie, 4. Auflage, Band VIII, Seiten 274 – 277 [1952]).

Als bevorzugte Beispiele für α-Hydroxynitrile der Formel (III) seien genannt: Acetoncyanhydrin, Methylethylketon-cyanhydrin, Cyclohexanon-cyanhydrin.

Als Verdünnungsmittel, die bei der Durchführung des erfindungsgemäßen Verfahrens eingesetzt werden können, kommen alle inerten organischen Lösungsmittel, die weder mit den Carbonsäureanhydriden noch den α-Hydroxycarbonsäurenitrilen (Cyanhydrinen) eine chemische Reaktion eingehen,

in Betracht. Solche Lösungsmittel sind beispielsweise die Xylole wie o-Xylol, Chlorbenzol, o-Dichlorbenzol, die Trichlorbenzole, Nitrobenzol, Tetramethylensulfon und Carbonsäurenitrile wie Acetonitril oder Propionitril. Als Verdünnungsmittel ist auch ein Überschuß an Carbonsäureanhydrid (II) bzw. insbesondere an $\alpha$-Hydroxynitril (III) geeignet. — Prinzipiell ist es jedoch auch möglich, die erfindungsgemäße Umsetzung ohne Verdünnungsmittel durchzuführen.

Die Reaktionstemperatur kann in einem relativ großen Bereich variiert werden. Im allgemeinen arbeitet man bei Temperaturen zwischen 250 und 600° C, vorzugsweise zwischen 300 und 500° C.

Die Reaktion wird im allgemeinen bei Normaldruck durchgeführt.

Bei der Durchführung des erfindungsgemäßen Verfahrens setzt man im allgemeinen auf 1 Mol Carbonsäureanhydrid (II) 0,7 bis 5 Mol, vorzugsweise 1 bis 4 Mol $\alpha$-Hydroxynitril (III) ein.

Die Aufarbeitung und Isolierung der Produkte erfolgt nach beendeter Umsetzung üblicherweise durch Destillation und gegebenenfalls Umkristallisation.

In einer besonderen Verfahrensvariante läßt sich die erfindungsgemäße Umsetzung auch kontinuierlich gestalten.

Die nach dem erfindungsgemäßen Verfahren herstellbaren Acylcyanide der Formel (I) sind weitgehend bekannt (vgl. zum Beispiel Thesing et al., Angew. Chem. 68, 425—435 [1956], ferner DE-OS 2 528 211, 2 614 240, 2 614 241, 2 614 242, 2 624 891, 2 642 140, 2 642 199, 2 708 182, 2 708 183, 2 717 075, 2 820 575); sie sind wertvolle Ausgangsstoffe z. B. zur Synthese von 1,2,4-Triazin-5-onen, welche hervorragende herbizide Eigenschaften besitzen (vgl. zum Beispiel DE-OS 2 224 161; DE-PS 1 795 784).

So läßt sich beispielsweise das 3-Methyl-4-amino-6-phenyl-1,2,4-triazin-5(4H)-on der Formel

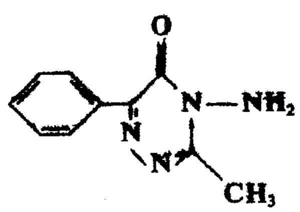

herstellen, indem man in einer ersten Stufe Benzoylcyanid in Gegenwart von konzentrierter Salzsäure mit Äthanol umsetzt und den dabei entstehenden Phenylglyoxylsäureethylester in einer zweiten Stufe mit Acetylhydrazin zur Reaktion bringt, wobei sich 1-Phenylglyoxylsäureethylester-2-acetylhydrazon bildet, das in einer dritten Stufe mit Hydrazinhydrat in Gegenwart von Pyridin in das obenerwähnte Endprodukt überführt wird.

Diese mehrstufige Synthese läßt sich formelmäßig wie folgt wiedergeben:

1. Stufe:

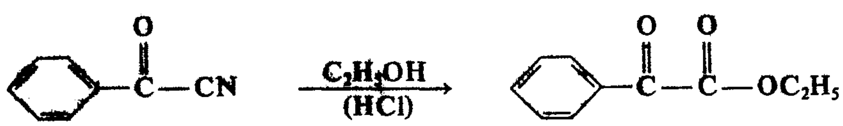


2. Stufe:

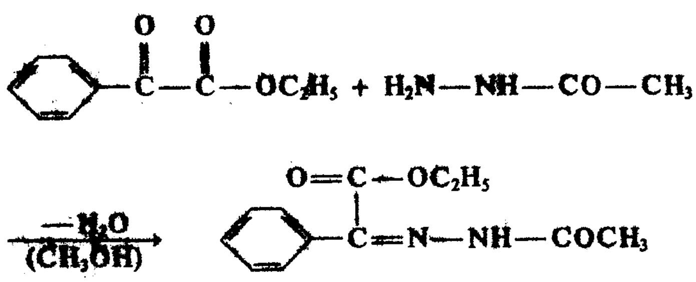


3. Stufe

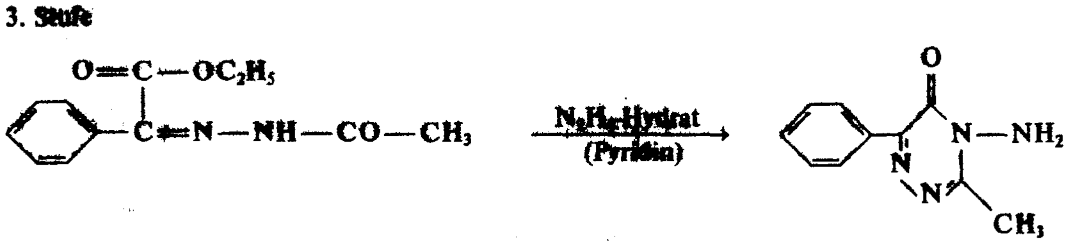

Das erfindungsgemäß herstellbare Pivaloylcyanid läßt sich nach bekannten Verfahren z. B. in den herbiziden Wirkstoff 3-Methylthio-4-amino-6-tert.-butyl-1,2,4-triazin-5-(4H)-on überführen (vgl. DE-OS 2 733 180, US-PS 4 175 188, ferner deutsche Patentanmeldungen P 3 002 203.8, P 3 003 541.7, P 3 009 043.8).

Das erfindungsgemäße Verfahren wird durch die nachfolgenden Herstellungsbeispiele veranschaulicht:

Herstellungsbeispiele

Beispiel 1

$(CH_3)_3C-CO-CN$

186 g Pivalinsäureanhydrid (1 Mol) und 255 g Acetoncyanhydrin (3 Mol) werden gemischt und innerhalb von drei Stunden durch ein mit Raschigringen gefülltes Glasrohr, das auf 450° C geheizt ist, getropft. Die Reaktionsprodukte werden in einer gekühlten Vorlage gesammelt und anschließend fraktioniert destilliert.

Ausbeute: 97,7 g Pivaloylcyanid (88% der Theorie); Siedepunkt: 124 – 126° C.

Das überschüssige Acetoncyanhydrin und das nichtumgesetzte Pivalinsäureanhydrid werden für den nächsten Ansatz verwendet.

Beispiel 2

113 g Benzoesäureanhydrid (0,5 Mol) und 127,5 g Acetoncyanhydrin (1,5 Mol) werden innerhalb einer Stunde durch ein auf 400° C geheiztes, mit Raschigringen gefülltes, 90 cm langes Duranglasrohr (Durchmesser 3,8 cm) getropft. Das erhaltene Reaktionsgemisch wird anschließend mit Waschbenzin aufgekocht; nach Abkühlung wird die ausgefallene Benzoesäure abfiltriert. Das Filtrat wird zunächst durch Abziehen der leicht flüchtigen Bestandteile eingeengt, dann fraktioniert destilliert.

Ausbeute: 49,1 g Benzoylcyanid (75% der Theorie); Schmelzpunkt: 32° C.

**Patentansprüche**

1. Verfahren zur Herstellung von Acylcyaniden der allgemeinen Formel

$$R^1-CO-CN \qquad (I)$$

in welcher

$R^1$ für geradkettiges oder verzweigtes Alkyl mit 1 – 4 C-Atomen, welches durch Alkoxy mit 1 – 4 C-Atomen, Carbalkoxy mit 1 – 4 C-Atomen in der Alkoxygruppe, Nitro, Cyano und/oder Halogen substituiert sein kann; für Cycloalkyl mit 5 – 6 Ring-C-Atomen, welches durch Alkyl, Alkoxy oder Carbalkoxy mit jeweils bis zu 4 C-Atomen, Nitro, Cyano und/oder Halogen substituiert sein kann; für Aryl, insbesonders Phenyl oder Naphthyl, welches durch Alkyl, Alkoxy oder Carbalkoxy mit jeweils bis zu 4 C-Atomen, Nitro und/oder Halogen substituiert sein kann, oder für 5- oder 6gliedrige heterocyclische Reste steht, die 1 bis 3 Heteroatome wie Sauerstoff, Schwefel und/oder Stickstoff im Ring enthalten können und außerdem mit einem Benzolring anelliert sein können,

dadurch gekennzeichnet, daß man Carbonsäureanhydride der allgemeinen Formel

$$R^1-CO-O-CO-R^1 \qquad (II)$$

in welcher

$R^1$ die oben angegebene Bedeutung hat,

mit α-Hydroxynitrilen der allgemeinen Formel

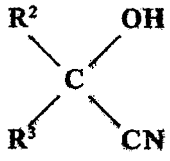

(III)

in welcher

$R^2$ und $R^3$ gleich oder verschieden sein können und für geradkettiges oder verzweigtes Alkyl mit 1–8 C-Atomen, für Cycloalkyl mit 5 oder 6 Ring-C-Atomen, welches durch Alkyl oder Carbalkoxy mit jeweils 1–5 C-Atomen substituiert sein kann, oder für Phenyl oder Naphthyl stehen, welches jeweils durch Alkyl, Halogenalkyl, Alkoxy oder Carbalkoxy mit jeweils bis zu 4 C-Atomen, Nitro und/oder Halogen substituiert sein kann,

gegebenenfalls in Gegenwart eines Verdünnungsmittels bei Temperaturen zwischen 250 und 600°C umsetzt.

2. Verfahren nach Anspruch 1, dadurch gekennzeichnet, daß man bei Temperaturen zwischen 300 und 500°C arbeitet.

3. Verfahren nach Anspruch 1, dadurch gekennzeichnet, daß auf 1 Mol Carbonsäureanhydrid (II) 0,7–5 Mol, vorzugsweise 1–4 Mol $\alpha$-Hydroxynitril (III) eingesetzt werden.

4. Verfahren nach Anspruch 1, dadurch gekennzeichnet, daß als Carbonsäureanhydride Pivalinsäureanhydrid oder Benzoesäureanhydrid eingesetzt werden.

5. Verfahren nach Anspruch 1, dadurch gekennzeichnet, daß als $\alpha$-Hydroxynitrile Acetoncyanhydrin, Methylethylketoncyanhydrin oder Cyclohexanon-cyanhydrin eingesetzt werden.

**Claims**

1. Process for the preparation of acyl cyanides of the general formula

$$R^1-CO-CN \quad (I)$$

in which

$R^1$ represents straight-chain or branched alkyl with 1–4 C atoms, which can be substituted by alkoxy with 1–4 C atoms, carbalkoxy with 1–4 C atoms in the alkoxy group, nitro, cyano and/or halogen; cycloalkyl with 5–6 ring C atoms, which can be substituted by alkyl, alkoxy or carbalkoxy with in each case up to 4 C atoms, nitro, cyano and/or halogen; aryl, in particular phenyl or naphthyl, which can be substituted by alkyl, alkoxy or carbalkoxy with in each case up to 4 C atoms, nitro and/or halogen, or 5-membered or 6-membered heterocyclic radicals which can contain 1 to 3 hetero-atoms such as oxygen, sulphur and/or nitrogen, in the ring, and can also be fused to a benzene ring,

characterised in that carboxylic acid anhydrides of the general formula

$$R^1-CO-O-CO-R^1 \quad (II)$$

in which

$R^1$ has the meaning indicated above,

are reacted with $\alpha$-hydroxynitriles of the general formula

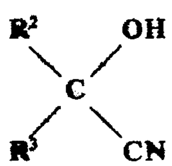

(III)

in which

$R^2$ and $R^3$ can be identical or different and represent straight-chain or branched alkyl with 1–8 C atoms, cycloalkyl with 5 or 6 ring C atoms, which can be substituted by alkyl or carbalkoxy with in each case 1–5 C atoms, or phenyl or naphthyl, each of which can be substituted by alkyl, halogenoalkyl, alkoxy or carbalkoxy with in each case up to 4 C atoms, nitro and/or halogen,

if appropriate in the presence of a diluent, at temperatures between 250 and 600°C.

2. Process according to Claim 1, characterised in that the reaction is carried out at temperatures between 300 and 500°C.

3. Process according to Claim 1, characterised in that 0.7–5 mols, preferably 1–4 mols, of $\alpha$-hydroxynitrile (III) are used per mol of carboxylic acid anhydride (II).

4. Process according to Claim 1, characterised in that pivalic anhydride or benzoic anhydride are employed as the carboxylic acid anhydrides.

5. Process according to Claim 1, characterised in that acetone cyanohydrin, methyl ethyl ketone cyanohydrin or cyclohexanone cyanohydrin are employed as the $\alpha$-hydroxynitriles.

**Revendications**

1. Procédé de production de cyanures d'acyle de formule générale

$$R^1-CO-CN \quad (I)$$

dans laquelle

$R^1$ est un groupe alkyle à chaîne droite ou ramifié ayant 1 à 4 atomes de carbone, qui peut être substitué par un radical alkoxy ayant 1 à 4 atomes de carbone, carbalkoxy ayant 1 à 4 atomes de carbone dans le groupe alkoxy, nitro, cyano et/ou halogéno; un groupe cycloalkyle ayant 5 ou 6 atomes de carbone dans le noyau, qui peut être substitué par un radical alkyle, alkoxy ou carbalkoxy ayant chacun jusqu'à 3 atomes de carbone, nitro, cyano et/ou halogéno; un groupe aryle, notamment phényle ou naphthyle qui peut être substitué par un radical alkyle, alkoxy ou carbalkoxy ayant chacun jusqu'à 4 atomes de carbone, nitro et/ou halogéno, ou des restes hétérocycliques pentagonaux ou hexagonaux qui peuvent contenir dans le noyau 1 à 3 hétéro-atomes tels qu'oxygène, soufre et/ou azote et peuvent en outre être condensés avec un noyau benzénique,

caractérisé en ce qu'on fait réagir à des températures comprises entre 250 et 600° C, éventuellement en présence d'un diluant, des anhydrides d'acides carboxyliques de formule générale

$$R^1-CO-O-CO-R^1 \quad (II),$$

dans laquelle

$R^1$ a la définition donnée ci-dessus,

avec des $\alpha$-hydroxynitriles de formule générale

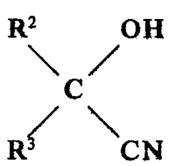

(III)

dans laquelle

$R^2$ et $R^3$ peuvent être égaux ou différents et représentent un groupe alkyle à chaîne droite ou ramifié ayant 1 à 8 atomes de carbone, cycloalkyle ayant 5 ou 6 atomes de carbone dans le noyau, qui peut être substitué par un radical alkyle ou carbalkoxy ayant chacun 1 à 5 atomes de carbone, ou un groupe phényle ou naphtyle qui peut être substitué dans chaque cas par un radical alkyle, halogénalkyle, alkoxy ou carbalkoxy ayant chacun jusqu' à 4 atomes de carbone, nitro et/ou halogéno.

2. Procédé suivant la revendication 1, caractérisé en ce qu'on opère à des températures comprises entre 300 et 500° C.

3. Procédé suivant la revendication 1, caractérisé en ce qu'on utilise par mole d'anhydride d'acide carboxylique (II) 0,7 à 5 moles, de préférence 1 à 4 moles d'$\alpha$-hydroxynitrile (III).

4. Procédé suivant la revendication 1, caractérisé en ce qu'on utilise comme anhydrides d'acides carboxyliques l'anhydride d'acide pivalique ou l'anhydride d'acide benzoïque.

5. Procédé suivant la revendication 1, caractérisé en ce qu'on utilise comme $\alpha$-hydroxynitriles la cyanhydrine de l'acétone, la cyanhydrine de la méthyléthyl-cétone la cyanhydrine de la cyclohexanone.